# EUROPEAN PATENT APPLICATION

(11) **EP 4 145 136 A1**
(43) Date of publication of application: **08.03.2023**
(21) Application number: 20934135.3
(22) Date of filing: 28.04.2020
(51) Int. Cl.: G01N 35/04, G01N 33/53

(54) **INCUBATION DEVICE AND AUTOMATIC ANALYSIS DEVICE**

(71) Applicant: Shenzhen Increcare Biotech Co., Ltd, Shenzhen, Guangdong 518055 (CN)
(72) Inventor: ZHANG, Zhen, Shenzhen, Guangdong 518055 (CN); HE, Taiyun, Shenzhen, Guangdong 518055 (CN); YU, Huaibo, Shenzhen, Guangdong 518055 (CN); YAO, Yanyi, Shenzhen, Guangdong 518055 (CN); LIU, Qilin, Shenzhen, Guangdong 518055 (CN)
(74) Representative: Inchingalo, Simona
(86) International application number: PCT/CN2020/087568
(87) International publication number: WO 2021/217443

(57) **Abstract**

The present invention relates to an incubation device and an automatic analysis device. The incubation device includes: an incubation unit (120) for incubating reaction containers (130) that contain a reactant or for buffering the cleaned and separated reaction containers (130), wherein the incubation unit (120) includes an incubation assembly (121) and an incubation driving assembly (122), the incubation driving assembly (122) is connected to the incubation assembly (121) so as to drive the incubation assembly (121) to move linearly along a third direction (30), and incubation positions (1211) for placing the reaction containers (130) are provided on the incubation assembly (121); and a transfer unit (110) for moving the reaction containers (130) into or out of the incubation unit (120), wherein the transfer unit (110) includes a pick-and-place assembly (112) and a pick-and-place driving assembly (111), the pick-and-place driving assembly (111) is connected to the pick-and-place assembly (112) so as to drive the pick-and-place assembly (112) to move along a first direction (10) and a second direction (20); and when the pick-and-place assembly (112) moves along the first direction (10), same able to move to above the incubation assembly (121), and the pick-and-place assembly (112) moves along the second direction (20) so as to move the reaction containers (130) into or out of the incubation positions (1211). The automatic analysis device includes the incubation device.

## Description

### Technical Field

The present invention relates to the technical field of in vitro diagnosis of medical devices, in particular to an incubation device and an automatic analysis device.

### Background

Automated immunoassay systems, such as chemiluminescence, electrochemiluminescence and flow fluorescence immunoassay analyzers, utilize the principles of self-luminescence and immunoreaction to associate an optical signal with the concentration of a to-be-tested substance and analyze the content of the to-be-tested substance in a sample, and are increasingly widely applied due to the characteristics of high sensitivity and specificity, wide linear range, and the like. With the increase in the amount of test samples, a clinical laboratory has higher and higher requirements for the volume (the smaller, the better) and the test flux (the larger, the better) of the immunoassay system. The test flux is understood as the output number of test results of test samples per unit time.

In the conventional technology, an incubation disc or a reaction disc is usually used as an incubation place of reaction containers that contain a reactant. In order to achieve high-flux testing, the size of the incubation disc needs to be increased to increase the number of incubation positions, but on one hand, the increase of the size of the incubation disc leads to overlarge control load and great difficulty of a driving control technology, so that the testing speed is low, and the high-flux testing cannot be achieved. On the other hand, the machining and manufacturing precision requirements are high and even difficult to achieve. Therefore, existing technical solutions either fail to achieve high-flux testing or lead to large size and high cost of the device.

### Summary

According to some embodiments of the present invention, an incubation device and an automatic analysis device are provided.

An incubation device includes: an incubation unit and a transfer unit.

The incubation unit is configured to incubate reaction containers that contain a reactant. The incubation unit includes an incubation assembly and an incubation driving assembly, the incubation driving assembly being connected to the incubation assembly so as to drive the incubation assembly to move linearly along a third direction, and incubation positions configured to place the reaction containers being provided on the incubation assembly.

The transfer unit is configured to move the reaction containers into or out of the incubation unit. The transfer unit includes a pick-and-place assembly and a pick-and-place driving assembly, the pick-and-place driving assembly being connected to the pick-and-place assembly so as to drive the pick-and-place assembly to move along a first direction and a second direction.

Herein, when the pick-and-place assembly moves along the first direction, same able to move to above the incubation assembly and the pick-and-place assembly moves along the second direction so as to move the reaction containers into or out of the incubation positions.

An automatic analysis device includes the above incubation device.

The automatic analysis device includes:
An incubation unit, the incubation unit is configured to incubate reaction containers that contain a sample and a reagent, the sample and the reagent being combined after incubation. The incubation unit includes an incubation assembly and an incubation driving assembly, the incubation driving assembly being connected to the incubation assembly so as to drive the incubation assembly to move linearly along a third direction, and incubation positions configured to place the reaction containers being provided on the incubation assembly.

A transfer unit, the transfer unit is configured to move the reaction containers into or out of the incubation unit. The transfer unit includes a pick-and-place assembly and a pick-and-place driving assembly, the pick-and-place driving assembly being connected to the pick-and-place assembly so as to drive the pick-and-place assembly to move along a first direction and a second direction.

A separation unit, the cleaning and separation unit is non-nested with the incubation unit and is configured to remove the unbound sample and reagent in the reaction containers.

Herein, when the pick-and-place assembly moves along the first direction, same able to move to above the incubation assembly and the pick-and-place assembly moves along the second direction so as to move the reaction containers into or out of the incubation positions.

The details of one or more embodiments of the present invention are proposed in the following drawings and descriptions. Other features, purposes, and advantages of the present invention become apparent from the specification, drawings, and claims.

### Brief Description of the Drawings

Fig. 1 is a schematic structural diagram of an incubation device in an embodiment of the present invention.
Figs. 2, 3 and 4 are schematic structural diagrams of a pick-and-place assembly in three embodiments of the present invention respectively.
Fig. 5 is a schematic structural diagram of an automatic analysis device in an embodiment of the present invention.
Fig. 6 is a flowchart of a one-step method of an immunoassay test in an embodiment of the present invention.
Fig. 7 is a flowchart of a delayed one-step method of an immunoassay test in an embodiment of the present invention.
Fig. 8 is a flowchart of a two-step method of an immunoassay test in an embodiment of the present invention.

Reference signs: 10. First direction; 20. Second direction; 30. Third direction; 110. Transfer unit; 111. Pick-and-place driving assembly; 111A. First driving piece; 111A1. First lifting driving piece; 111A2. Second lifting driving piece; 111A3. Third lifting driving piece; 111B. Second driving piece; 111C. First guide rail; 111C1. First lifting guide rail; 111C2. Second lift guide rail; 111C3. Third lifting guide rail; 111D. Second guide rail; 111E. First transmission assembly; 111E1. First lifting transmission assembly; 111E2. Second lifting transmission assembly; 111E3. Third lifting transmission assembly; 111J. Second transmission assembly; 111G. Fixed plate; 111H1. First switching assembly; 111H2. Second switching assembly; 111H3. Third switching assembly; 111I. Third driving piece; 111F. Third transmission assembly; 112. Pick-and-place assembly; 112A. First gripper; 112B. Second gripper; 112C. Third gripper; 120. Incubation unit; 121. Incubation assembly; 1211. Incubation position; 122. Incubation driving assembly; 130. Reaction container; 210. Measuring unit; 220. Measuring plate; 310. Cleaning and separation unit; 311. Rotary cleaning plate; 312. Magnetic device; 313. Cleaning and separation position.

### Detailed Description of the Embodiments

In order to make the purposes, technical solutions and advantages of the present invention clearer, the present invention will be further described below in detail in conjunction with the accompanying drawings and embodiments. It is to be understood that the specific embodiments described herein are only used to illustrate the present invention, but are not intended to limit the present invention.

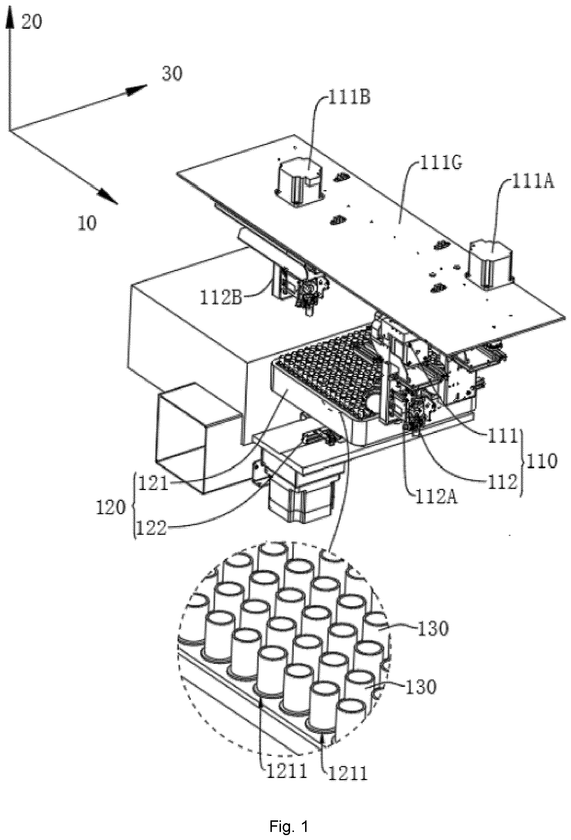

Referring to Fig. 1, which is a schematic structural diagram of an incubation device in an embodiment of the present invention. The incubation device is configured for an immunoassay test process.

An immunoassay test is a quantitative or qualitative test of a to-be-tested target substance, such as an antigen and an antibody contained in a blood sample. The immunoassay test is generally divided into a one-step method, a delayed one-step method and a two-step method according to a test principle and mode.

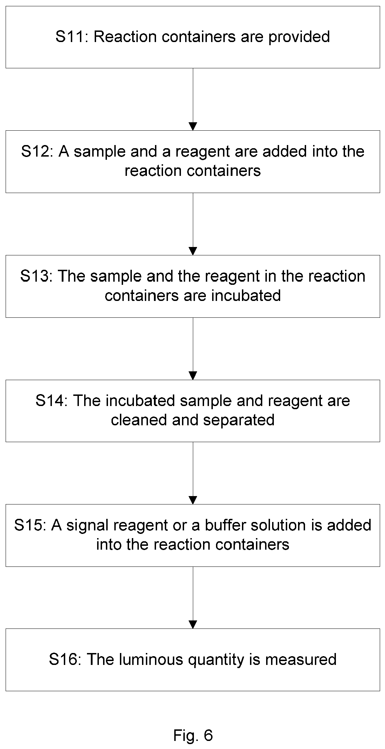

As shown in Fig. 6, which is a flowchart of a one-step method of an immunoassay test in an embodiment of the present invention. The one-step method mainly includes the following steps.

At S11, reaction containers 130 are provided.

At S12, a sample and a reagent are added into the reaction containers 130. Herein, different samples and reagents are correspondingly selected according to different test items. According to the different samples, reagents and test methods, some test items also require uniform mixing of the sample and the reagent in the reaction containers 130.

At S13, the sample and the reagent in the reaction containers 130 are incubated. Incubation is generally a process of reacting the sample with the reagent in the reaction containers 130 in a stable temperature environment. Usually, after the reaction, the reaction containers 130 contain a magnetic particle complex formed by the sample and the reagent, and the reaction containers 130 also contain the unreacted sample and reagent. The incubation time is usually 3-60 minutes.

At S14, the incubated sample and reagent are cleaned and separated. Cleaning and separation generally refers to magnetic trapping of magnetic particles in the reaction containers 130 while removing the unreacted sample and reagent.

At S16, the luminous quantity is measured.

In some immunoassay tests, S15 is also included between S14 and S16.

At S15, a signal reagent or a buffer solution is added into the reaction containers 130. Some immunoassay tests require incubation after the addition of the signal reagent. Signal incubation is usually performed in a stable temperature environment for 1-10 minutes. Signal incubation is able to enhance the luminous quantity or prepare for the generation of a light emitting signal. In some immunological tests, the first signal reagent is first added to prepare for signal generation, and then the luminous quantity is measured when the second signal reagent is added in S16. In some immunological tests, the buffer solution is added to recover the cleaned and separated magnetic particle complex, and the recovered magnetic particle complex is moved to a measuring device for measurement. Herein, the incubation device shown in Fig. 1 is mainly configured to complete S13 of the above one-step method. In some embodiments, the incubation device is configured to buffer the cleaned and separated reaction containers 130. For example, to buffer the reaction containers 130 added with the signal reagent or the buffer solution in S15 of the above one-step method.

As shown in Fig. 1, the incubation device includes an incubation unit 120 and a transfer unit 110. The transfer unit 110 is configured to move the reaction containers 130 into or out of the incubation unit 120, the incubation unit 120 is configured to incubate the reaction containers 130 that contain the reactant, and the reactant usually includes the sample and the reagent. In some embodiments, the incubation unit 120 is configured to buffer the cleaned and separated reaction containers 130.

As shown in Fig. 1, specifically, the incubation unit 120 includes an incubation assembly 121 and an incubation driving assembly 122, the incubation assembly 121 being connected to the incubation driving assembly122 so as to drive the incubation assembly 121 to move linearly along a third direction 30. Incubation positions 1211 are provided on the incubation assembly 121, and the reaction containers 130 are placed in the incubation positions 1211. The transfer unit 110 includes a pick-and-place assembly 112 and a pick-and-place driving assembly 111, the pick-and-place driving assembly 111 being connected to the pick-and-place assembly 112, and the pick-and-place driving assembly 111 driving the pick-and-place assembly 112 to move along a first direction 10 and a second direction 20. Herein, when the pick-and-place assembly 112 moves along the first direction 10, same moves to above the incubation assembly 121, and the pick-and-place assembly 112 moves along the second direction 20 so as to move the reaction containers 130 into or out of the incubation positions 1211.

It is to be noted that the "and" in which the pick-and-place driving assembly 111 drives the pick-and-place assembly 112 to move along the first direction 10 and the second direction 20 is not a limit in time or order, that is, it does not mean that the two are "at the same time", but means that the two are "both... and..." functionally, that is, the pick-and-place driving assembly 111 drives the pick-and-place assembly112 to move along either the first direction or the second direction 20. Specifically, the pick-and-place driving assembly 111 drive the pick-and-place assembly 112 to move along the first direction 10 first and then along the second direction 20, for example, when the pick-and-place assembly 112 moves one reaction container 130 into the incubation assembly 121; move along the second direction 20 and then along the first direction 10, for example, when the pick-and-place assembly 112 moves one reaction container 130 out of the incubation assembly 121; and move along the first direction 10 and the second direction 20 at the same time, for example, when the pick-and-place driving assembly 111 is reset. Usually, the application is in the first two cases, that is, the two are executed in sequence. The first direction 10, the second direction 20 and the third direction 30 include positive and negative directions, and during specific movement, the assembly able to move along the positive direction or the negative direction of the direction. For example, the incubation driving assembly121 drives the incubation assembly 121 to move linearly along the third direction 30, and the incubation driving assembly 121 able to move linearly along the positive direction or the negative direction of the third direction 30.

As shown in Fig. 1, in one embodiment, the first direction 10 and the third direction 30 are horizontal directions, and the second direction 20 is a vertical direction. Specifically, the incubation driving assembly 122 drives the incubation assembly 121 to move linearly along the third direction 30, the pick-and-place driving assembly 111 drives the pick-and-place assembly 112 to move linearly along the first direction 10, and when the pick-and-place assembly 112 moves to above the incubation assembly 121, the pick-and-place driving assembly 111 drives the pick-and-place assembly 112 to lift along the second direction 20, thereby moving the reaction containers 130 into or out of the incubation positions 1211.

In one embodiment, the first direction 10 and the third direction 30 are horizontal directions, the second direction 20 is a vertical direction, and the first direction 10 and the second direction 20 are perpendicular. For example, the first direction 10 is an X-axis direction, the third direction 30 is a Y-axis direction, and the second direction 20 is a Z-axis direction.

In one embodiment, the incubation positions 1211 are arranged in a matrix on the incubation assembly 121 (That is, a multi-row and multi-column arrangement form). The incubation assembly 121 is of a cuboid-like block structure. A plurality of rows of incubation positions 1211 are arranged along the third direction 30, and a plurality of columns of incubation positions 1211 are arranged along the first direction 10. Further, the difference between the number of rows and the number of columns of the incubation positions 1211 does not exceed 5. In this way, the incubation assembly 121 and the pick-and-place assembly 112 are more reasonable in division of labor and cooperation, and the problems that the moving travel of one assembly in the movement direction is too long, the moving travel of the other assembly in the movement direction is too short, and the like are avoided, so that the transfer efficiency of the reaction containers 130 is improved on the whole. On the other hand, the sizes of the transfer unit 110, the incubation unit 120 and the whole machine are reduced, and the reaction efficiency per unit area is improved. The number of the incubation positions 1211 is not smaller than 150, and continuous testing of the incubation device of the application is guaranteed. For example, the incubation positions 1211 are uniformly arranged on the incubation assembly 121, and fifteen rows and fifteen columns of incubation positions 1211 are arranged on the incubation assembly 121. Since the incubation positions 1211 are arranged in a matrix on the incubation assembly 121, the incubation positions 1211 are uniformly distributed on the incubation assembly 121.

At least one access station is provided on the incubation device along the third direction 30 for the transfer unit 110 to move the reaction container 130 into the incubation assembly 121 and move the reaction container 130 out of the incubation assembly 121. It is to be noted that the access station is an absolute position, configured to identify the absolute coordinate position at which the assembly works or performs a task, and does not move along with the movement of the incubation unit 120 and the transfer unit 110. In particular, the access station of the incubation device is located on a straight line where a moving trajectory of the pick-and-place assembly 112 along the first direction 10 is located. Further, the number of access stations of the incubation device is equal to the number of non-coincident moving trajectories of the pick-and-place assembly 112 along the first direction 10. Since the incubation positions 1211 are arranged in a matrix on the incubation assembly 121, the incubation unit 120 is provided with a plurality of rows of incubation positions 1211 along the third direction 30 and a plurality of columns of incubation positions 1211 along the first direction 10. The moving trajectory of the transfer unit 110 along the first direction 10 at least covers the distance between the head and tail columns of the incubation positions 1211 on the incubation assembly 121 along the first direction 10. The incubation driving assembly 122 drives the incubation assembly 121 to move linearly along the third direction 30 so as to convey any row of incubation positions 1211 on the incubation assembly 121 to the access station, and the pick-and-place driving assembly 111 drives the pick-and-place assembly 112 to linearly move along the first direction 10 so as to drive the pick-and-place assembly 112 to above a certain row and any column of incubation positions 1211 located on the incubation assembly 121 of the access station, thereby traversing the incubation positions 1211 on the incubation assembly 121.

In one embodiment, the incubation assembly 121 is slidably connected to the incubation driving assembly 122. In this way, the driving load of the incubation driving assembly 122 is reduced to facilitate high speed movement of the incubation assembly 121. In one embodiment, the incubation driving assembly 122 is located below the incubation assembly 121, which makes full use of the size in the vertical direction, further reduces the size of the incubation device along the horizontal direction, and facilitate reduction of the sizes of the incubation device and the automatic analysis device. On the other hand, the incubation driving assembly 122 is located below the incubation assembly 121, the incubation positions 1211 are uniformly distributed on the incubation assembly 121, and the problem that the incubation positions cannot be distributed on the incubation assembly due to the fact that a driving assembly is installed on the incubation assembly in the conventional art is solved.

The pick-and-place driving assembly 111 drives the pick-and-place assembly 112 to only move along the first direction 10 and the second direction 20 but not along the third direction, so that the transfer structure is simplified, the driving load is reduced, and the reaction container 130 is transferred more efficiently.

In the conventional technology, the incubation positions 1211 are annularly arranged on the periphery of an incubation disc, and the incubation disc rotates around its own rotating shaft to achieve pick-and-place of the reaction containers 130 at different incubation positions 1211. The incubation positions 1211 are usually only arranged in the outer ring and the middle of the incubation disc is usually empty, thereby leading in a smaller number of incubation positions 1211 on the incubation disc. In order to achieve high-flux testing, the number of incubation positions 1211 needs to be increased by increasing the size of the incubation disc, but increasing the size of the incubation disc leads to overlarge control load and large difficulty of the driving control technology on one hand, and on the other hand, the incubation positions 1211 of the incubation disc have high machining precision requirements and great machining challenges on the incubation disc. In addition, in order to achieve incubation, the size and precision requirements of an incubation pot wrapping the incubation disc are increased, a large-size pot body is difficult to machine in the existing manufacturing process, and the cost is very high even if the large-size pot body is manufactured.

In the embodiment of the present invention, the incubation positions 1211 are arranged on the incubation unit 120 to mainly complete the incubation step in S13, so that more incubation positions 1211 is arranged on the premise of ensuring that the size of the incubation assembly 121 is small, and the size of the incubation device is small. Both the incubation unit 120 and the transfer unit 110 able to move independently, and the movement flexibility is high. Through the linear motion of the incubation unit 120 along the third direction 30, the transfer unit 110 only needs to move linearly along the first direction 10 to traverse all the incubation positions 1211 on the incubation assembly 121, and the incubation unit 120 and the transfer unit 110 able to move at a high speed in a coordinated manner, so that the efficiency of traversing all the incubation positions 1211 on the incubation assembly 121 and the reaction containers 130 thereon is improved, and the high-flux testing is realized. As a contrast, if the incubation unit 120 is fixed, the transfer unit 110 moves linearly along both the first direction 10 and the third direction 30, although all the incubation positions 1211 on the incubation assembly 121 be traversed, due to the large number of incubation positions and limitation of the large range of motion of the transfer unit 110, complex driving and limited speed, all the incubation positions 1211 on the incubation assembly 121 is not traversed at a high speed, high-flux testing cannot be achieved.

In the one-step method, the incubation assembly 121 moves linearly along the third direction 30 so as to convey a row of incubation positions where an empty incubation position 1211 is located to the access position of the incubation device, the pick-and-place assembly 112 moves linearly along the first direction 10 to above the empty incubation position 1211, and the pick-and-place assembly 112 lifts along the second direction 20 to move the reaction container 130 filled with the sample and the reagent into the empty incubation position 1211. After the sample and the reagent in the reaction container 130 are incubated at the incubation position 1211 on the incubation assembly 121 for a set time, the incubation assembly 121 moves linearly along the third direction 30 so as to convey the reaction container 130 that needs to be cleaned and separated after incubation for a set time to the access position of the incubation device, the pick-and-place assembly 112 moves linearly along the first direction 10 to above the incubation position 1211 where the reaction container 130 is located, and the pick-and-place assembly 112 lifts along the second reaction 20 to move the reaction container 130 out.

In some embodiments, incubation assembly 121 is configured to buffer the cleaned and separated reaction container 130. The incubation assembly 121 moves linearly along the third direction 30 so as to convey a row of incubation positions where an empty incubation position 1211 is located to the access position of the incubation device, the pick-and-place assembly 112 moves linearly along the first direction 10 to above the empty incubation position 1211, and the pick-and-place assembly 112 lifts along the second direction 20 to move the reaction container 130 filled with the signal reagent or the buffer solution into the incubation position 1211. If the cleaned and separated reaction container 130 that contains the signal reagent or the buffer solution needs to be incubated, incubation is achieved while the reaction container 130 is buffered on the incubation assembly 121. For example, after the reaction container 130 that contains the signal reagent or the buffer solution is buffered and incubated for a set time on the incubation assembly 121, the incubation assembly 121 moves linearly along the third direction so as to convey the reaction container 130 that contains the signal reagent and needs to measure the luminous quantity to the access position of the incubation device, the pick-and-place assembly 112 moves linearly along the first direction 10 to above the incubation position 1211 where the reaction container 130 is located, and the pick-and-place assembly 112 lifts along the second direction 20 to move he reaction container 130 out. If the reaction container 130 that contains the signal reagent or the buffer solution does not need to be incubated, after the reaction container 130 is buffered on the incubation assembly 121, the incubation assembly 121 moves linearly along the third direction so as to convey the cleaned and separated reaction container 130 that contains the signal reagent or the buffer solution and needs to measure the luminous quantity to the access position of the incubation device, and the transfer unit 110 moves the reaction container 130 out. From the above, for the reaction container 130 that contains the signal reagent or the buffer solution and does not need to be incubated, the incubation assembly 121 is configured to transfer the reaction container 130.

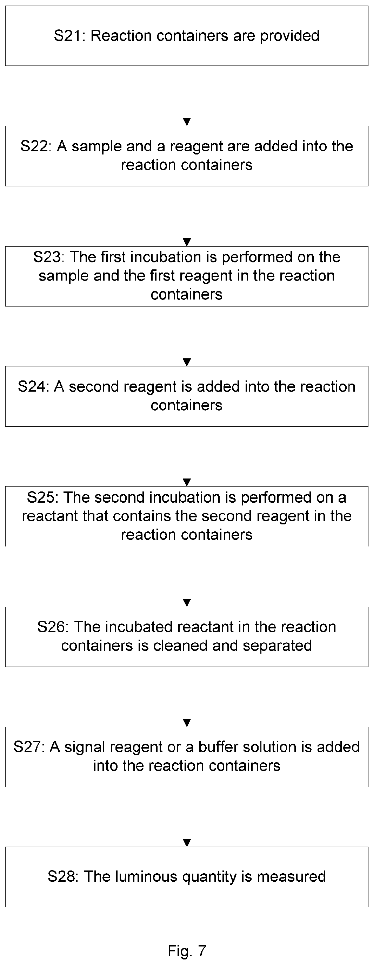

As shown in Fig. 7, Fig. 7 is a flowchart of a delayed one-step method of an immunoassay test in an embodiment of the present invention. The main difference between the delayed one-step method and the aforementioned one-step method is S22-S25 which are briefly introduced below, and the rest of the steps are the same as or similar to those of the one-step method and will not be elaborated herein. The process of S22-S25 of the delayed one-step method is as follows.

At S22, a sample and a first reagent are added into the reaction container 130. Herein, different samples and first reagents are correspondingly selected according to different test items. According to the different samples, first reagents and test methods, some test items also need to uniformly mix the sample and the first reagent in the reaction container 130.

At S23, the first incubation is performed on the sample and the first reagent in the reaction container 130. Incubation is usually a process of reacting the sample with the first reagent in the reaction container 130 in a stable temperature environment. The incubation time is usually 3-60 minutes.

At S24, a second reagent is added into the reaction container 130. Herein, different second reagents are correspondingly selected according to different test items. According to the different second reagents and test methods, some test items also need to uniformly mix a reactant that contains the second reagent in the reaction container 130.

At S25, the second incubation is performed on the reactant that contains the second reagent in the reaction container 130.

In the delayed one-step test, the incubation assembly 121 moves linearly along the third direction 30 so as to convey the empty incubation position 1211 to the access position of the incubation device, and the transfer unit 110 moves the reaction container 130 filled with the sample and the first reagent into the incubation position 1211. After the first incubation is performed on the sample and the first reagent in the reaction container 130 for a set time, the incubation assembly 121 moves linearly along the third direction 30 so as to convey the reaction container 130 that needs to be filled with the second reagent to the access position of the incubation device, and the transfer unit 110 moves the reaction container 130 out. The incubation assembly 121 moves linearly along the third direction 30 so as to convey the empty incubation position 1211 to the access position of the incubation device, and then the transfer unit 110 moves the reaction container 130 filled with the second reagent into the incubation position 1211. After the second incubation is performed on the reactant that contains the second reagent in the reaction container 130 for a set time, the incubation assembly 121 moves linearly along the third direction 30 so as to convey the reaction container 130 that needs to be cleaned and separated after the second incubation for a set time to the access position of the incubation device, and the transfer unit 110 moves the reaction container 130 out. The subsequent steps of the delayed one-step test and the role and function of the incubation device in the subsequent steps are the same as or similar to those of the one-step method and will not be elaborated herein.

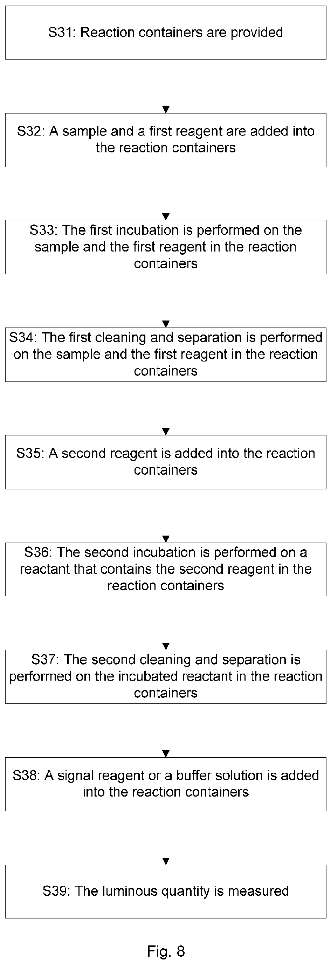

As shown in Fig. 8, Fig. 8 is a flowchart of a two-step method of an immunoassay test in an embodiment of the present invention. The two-step method mainly includes the following steps that: the main difference between the two-step method and the aforementioned delayed one-step method is S34-S37 which are briefly introduced below, and the rest of the steps are the same as or similar to those of the one-step method and will not be elaborated herein. The process of S34-S37 of the two-step method is as follows.

At S34, a sample and a first reagent in the reaction container 130 are cleaned and separated for the first time.

At S35, a second reagent is added into to the reaction container 130. Herein, different second reagents are correspondingly selected according to different test items. According to the different second reagents and test methods, some test items also need to uniformly mix a reactant that contains the second reagent in the reaction container 130.

At S36, the second incubation is performed on the reactant that contains the second reagent in the reaction container 130.

At S37, the second cleaning and separation is performed on the incubated reactant in the reaction container 130.

In the two-step method, the incubation assembly 121 moves linearly along the third direction 30 so as to convey the empty incubation position 1211 to the access position of the incubation device, and the transfer unit 110 moves the reaction container 130 filled with the sample and the first reagent into the incubation position 1211. After the first incubation is performed on the reactant in the reaction container 130 for a set time, the incubation assembly 121 moves linearly along the third direction 30 so as to convey the reaction container 130 that needs the first cleaning and separation after the incubation for a set time to the access position of the incubation device, and the transfer unit 110 moves the reaction container 130 out. When the pick-and-place assembly 112 only includes one gripper, the reaction container 130 after the first cleaning and separation is directly transferred by the transfer unit 110 to be filled with the sample and the second reagent. When the pick-and-place assembly 112 includes at least two grippers, the reaction container 130 that needs to be filled with the second reagent after the first cleaning and separation is buffered and transferred by the incubation assembly 121. When transferring by the incubation assembly 121, the moving travel of each gripper along the first direction 10 is reduced, and the space setting and working time of each gripper is more flexible. When buffering and transferring by the incubation assembly 121, the incubation assembly 121 moves linearly along the third direction 30 so as to convey the empty incubation position 1211 to the access position of the incubation device, and then one gripper of the transfer unit 110 moves the cleaned reaction container 130 into the incubation position 1211. The incubation assembly 121 moves linearly along the third direction of 30 so as to convey the reaction container 130 that needs to be filled with the second reagent on the incubation position 1211 to the access position of the incubation device, and another gripper of the transfer unit 110 moves the reaction container 130 out. The incubation assembly 121 moves linearly along the third direction 30 so as to convey the empty incubation position 1211 to the access position of the incubation device, and then the transfer unit 110 moves the reaction container 130 filled with the second reagent into the incubation position 1211. After the second incubation is performed on the reactant in the reaction container 13 for a set time, the incubation assembly 121 moves linearly along the third direction 30 so as to convey the reaction container 130 that needs the second cleaning and separation after incubation for a set time to the access position of the incubation device, and the transfer unit 110 moves the reaction container 130 out. The subsequent steps of the two-step test and the role and function of the incubation device in the subsequent steps are the same or similar to those of the delayed one-step method and will not be elaborated herein.

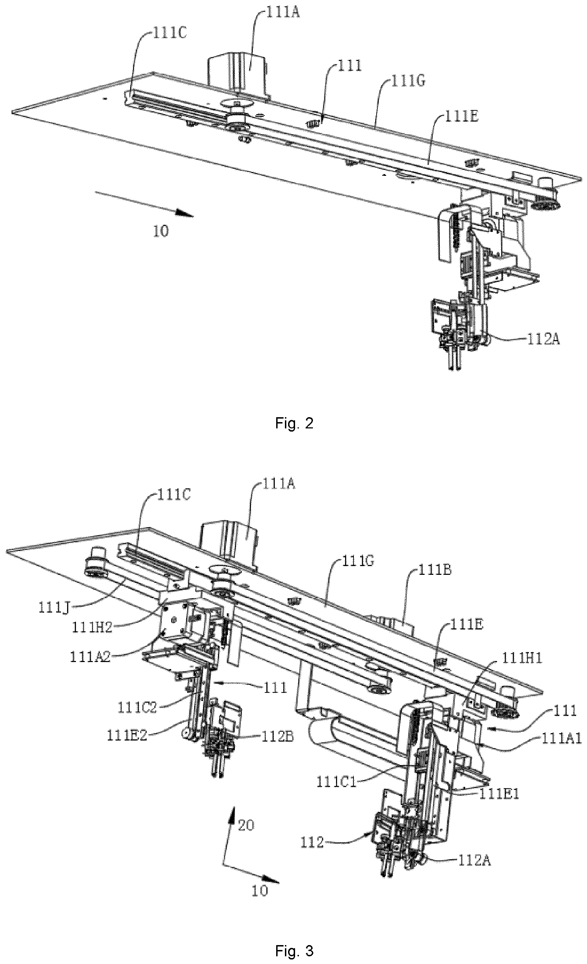

Figs. 2, 3, and 4 are schematic structural diagrams of the pick-and-place assembly 112 in three embodiments of the application respectively.

In one embodiment, as shown in Figs. 1 and 2, the pick-and-place assembly 112 includes a first gripper 112A, the pick-and-place driving assembly 111 being connected to the first gripper 112A, and the pick-and-place driving assembly 111 driving the first gripper 112A to move along the first direction 10 and the second direction 20. The pick-and-place driving assembly 111 includes a fixed plate 111G, a first guide rail 111C, a first driving piece 111A, a first transmission assembly 111E, a first switching assembly 111H1, a first lifting guide rail 111C1, a first lifting driving assembly 111A1, and a first lifting transmission assembly 111E1.

The first guide rail 111C is fixedly connected to the fixed plate 111G, the first guide rail 111C extends along the first direction 10, and the first switching assembly 111H1 is slidably connected to the first guide rail 111C. The first driving piece 111A is fixedly connected to the fixed plate 111G, and the first driving piece 111A is a motor.

The first driving assembly 111E is connected to the output end of the first driving assembly 111A and the first switching assembly 111H1 respectively, so that the first driving assembly 111A drives the first switching assembly 111H1 to slide along the first guide rail 111C.

The first lifting guide rail 111C1 is fixedly connected to the first switching assembly 111H1, the first lifting guide rail 111C1 extends along the second direction 20, and the first gripper 112A is slidably connected to the first lifting guide rail 111C1. The first lifting driving piece 111A1 is fixedly connected to the first switching assembly 111H1, and the first lifting driving piece 111A1 is a motor. The first lifting transmission assembly 111E1 is arranged on the first switching assembly 111H 1. The first lifting driving assembly 111E1 is connected to the output end of the first lifting driving piece 111A1 and the first gripper 112A.

In one embodiment, as shown in Fig. 3, the pick-and-place assembly 112 includes a first gripper 112A and a second gripper 112B. The pick-and-place driving assembly 111 includes a fixed plate 111G, a first guide rail 111C, a first driving piece 111A, a second driving piece 111B, a first transmission assembly 111E, a second transmission assembly 111J, a first switching assembly 111H1, a second switching assembly 111H2, a first lifting guide rail 111C1, a second lifting guide rail 111C2, a first lifting driving piece 111A1, a second lifting driving piece 111A2, a first lifting transmission assembly 111E1, and a second lifting transmission assembly 111E2.

The first switching assembly 111H1 is slidably connected to the first guide rail 111C, and the second switching assembly 111H2 is also slidably connected to the first guide rail 111C.

The first transmission assembly 111E is arranged on the fixed plate 111G, and the first transmission assembly 111E is connected to the output end of the first driving piece 111A and the first switching assembly 111H1 respectively, so that the first driving piece 111A drives the first switching assembly 111H1 to slide along the first guide rail 111C.

The second transmission assembly 111J and the second driving piece 111B are both arranged on the fixed plate 111G, and the second transmission assembly 111J is connected to the output end of the second driving piece 111B and the second switching assembly 111H2 respectively, so that the second driving piece 111B drives the second switching assembly 111H2 to slide along the first guide rail 111C.

The first lifting guide rail 111C1 is fixedly connected to the first switching assembly 111H1, the first lifting guide rail 111C1 extends along the second direction 20, and the first gripper 112A is slidably connected to the first lifting guide rail 111C1. The first lifting driving piece 111A1 is fixedly connected to the first switching assembly 111H1, and the first lifting driving piece 111A1 is a motor. The first lifting transmission assembly 111E1 is arranged on the first switching assembly 111H1. The first lifting driving assembly 111E1 is connected to the output end of the first lifting driving piece 111A1 and the first gripper 112A respectively.

The second lifting guide rail 111C2 is fixedly connected to the second switching assembly 111H2, the second lifting guide rail 111C2 extends along the second direction 20, and the second gripper 112C is slidably connected to the second lifting guide rail 111C2. The second lifting driving piece 111A2 is fixedly connected to the second switching assembly 111H2, and the second lifting driving piece 111A2 is a motor. The second lifting transmission assembly 111E2 is arranged on the second switching assembly 111H2. The second lifting transmission assembly 111E2 is connected to the output end of the second lifting driving piece 111A2 and the second gripper 112B respectively.

In the embodiment shown in Fig. 3, the first switching assembly 111H1 and the second switching assembly 111H2 both slide along the first guide rail 111C with at least one section of overlapped trajectory, so that both first gripper 112A and second gripper 112B able to move to above the incubation assembly 121. Further, there is at least one section of overlapped motion trajectory of the first gripper 112A and the second gripper 112B between the head and tail columns of the incubation positions of the incubation assembly 121. Specifically, as shown in Fig. 1, the incubation assembly 121 moves along the third direction 30. Incubation positions 1211 arranged in a matrix are provided on the incubation assembly 121, that is, the plurality of incubation positions 1211 are arranged along the first direction 10 (called a column), and the plurality of incubation positions 1211 are arranged along the third direction 30 (called a row). The incubation assembly 121 moves linearly along the third direction 30, and when the first gripper 112A moves along the first direction 10, same able to move to above all the incubation positions 1211 arranged along the first direction 10 on the incubation assembly 121. The incubation assembly 121 moves linearly along the third direction 30, and when the second gripper 112B moves along the first direction 10, same able to move to above all the incubation positions 1211 arranged along the first direction 10 on the incubation assembly 121. In the embodiment, the straight lines where the trajectories of the first gripper 112A and the second gripper 112B are located coincide. Since the number of access stations of the incubation device is equal to the number of non-coincident motion trajectories of the pick-and-place assembly 112 along the first direction 10, the number of access stations of the incubation device in the embodiment is one. That is, in the embodiment, the incubation device is provided with one access station. In the embodiment, the incubation assembly 121 only needs to move along the third direction 30, but does not need to move along the first direction 10, and the pick-and-place assembly 112 does not need to move along the third direction 30, so that the first gripper 112A and the second gripper 112B pick and place the reaction containers 130 in all the incubation positions 1211 on the incubation assembly 121. In addition, the incubation device is provided with only one access station, and the incubation assembly 121 does not need to move to a plurality of access stations when moving along the third direction 30, which further reduces the moving travel of the incubation assembly 121, improves the efficiency of the incubation assembly 121 in treating the reaction container 130, saves the operation space of the incubation assembly 121, and reduces the size of the whole machine.

During operation, only one gripper moves the reaction container 130 in and out each time the incubation assembly 121 moves linearly along the third direction 30 to the access position to stop. The first gripper 112A and the second gripper 112B move the reaction container 130 into and out of the incubation assembly 121 in a labor and time division manner. Specifically, the first gripper 112A moves the reactor that needs to be cleaned and separated out of the incubation assembly 121 and moves the cleaned and separated reaction container into the incubation assembly 121. The second gripper 112B moves the reactor that needs to be filled with the second reagent out of the incubation assembly 121, moves the reaction container that needs to be incubated after being filled with the reagent into the incubation assembly 121, and moves the reactor that needs to be measured after being moved and buffered by the incubation assembly 121 out of the incubation assembly 121. The first gripper 112A moves the reaction container 130 in and out and the second gripper 112B moves the reaction container 130 in and out at different times, that is, only one gripper moves the reaction container 130 in and out each time the corresponding incubation assembly 121 moves linearly along the third direction 30 to the access position.

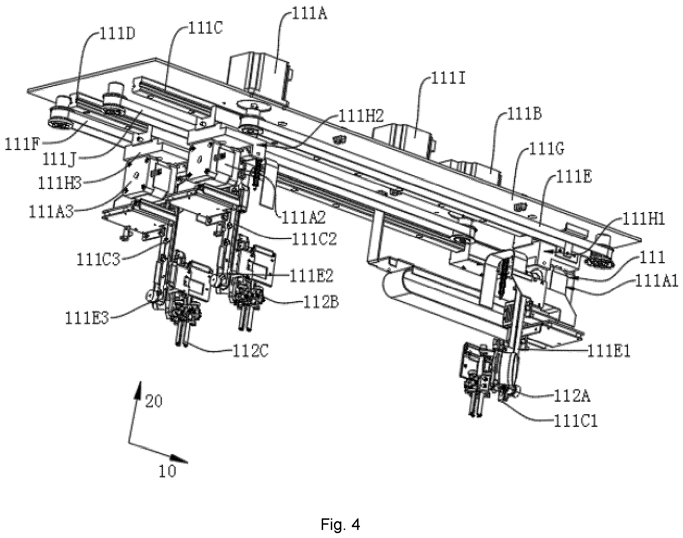

In one embodiment, as shown in Fig. 4, the pick-and-place assembly 112 includes a first gripper 112A, a second gripper 112B, and a third gripper 112C. The pick-and-place driving assembly 111 includes a fixed plate 111G, a first guide rail 111C, a second guide rail 111D, a first driving piece 111A, a second driving piece 111B, a third driving piece 111I, a first transmission assembly 111E, a second transmission assembly 111J, a third transmission assembly 111F, a first switching assembly 111H1, a second switching assembly 111H2, a third switching assembly 111H3, a first lifting guide rail 111C1, a second lifting guide rail 111C2, a third lifting guide rail 111C3, a first lifting driving piece 111A1, a second lifting driving piece 111A2, a third lifting driving piece 111A3, a first lifting transmission assembly 111E1, a second lifting transmission assembly 111E2, and a third lifting transmission assembly111E3.

Herein, the first guide rail 111C is parallel to the second guide rail 111D.

The first switching assembly 111H1 is slidably connected to the first guide rail 111C, the second switching assembly 111H2 is also slidably connected to the first guide rail 111C, and the third switching assembly 111H3 is slidably connected to the second guide rail 111D.

The first transmission assembly 111E is arranged on the fixed plate 111G, and the first transmission assembly 111E is connected to the output end of the first driving piece 111A and the first switching assembly 111H1 respectively, so that the first driving piece 111A drives the first switching assembly 111H1 to slide along the first guide rail 111C.

The second transmission assembly 111J and the second driving piece 111B are both arranged on the fixed plate 111G, and the second transmission assembly 111J is connected to the output end of the second driving piece 111B and the second switching assembly 111H2 respectively, so that the second driving piece 111B drives the second switching assembly 111H2 to slide along the first guide rail 111C.

The third transmission assembly 111F and the third driving piece 111I are both arranged on the fixed plate 111G, and the third transmission assembly 111F is connected to the output end of the third driving piece 111I and the third switching assembly 111H3 respectively, so that the third driving piece 111I drives the third switching assembly 111H3 to slide along the first guide rail 111C.

The first lifting guide rail 111C1 is fixedly connected to the first switching assembly 111H1, the first lifting guide rail 111C1 extends along the second direction 20, and the first gripper 112A is slidably connected to the first lifting guide rail 111C1. The first lifting driving piece 111A1 is fixedly connected to the first switching assembly 111H1, and the first lifting driving piece 111A1 is a motor. The first lifting transmission assembly 111E1 is arranged on the first switching assembly 111H1. The first lifting driving assembly 111E1 is connected to the output end of the first lifting driving piece 111A1 and the first gripper 112A respectively.

The second lifting guide rail 111C2 is fixedly connected to the second switching assembly 111H2, the second lifting guide rail 111C2 extends along the second direction 20, and the second gripper 112B is slidably connected to the second lifting guide rail 111C2. The second lifting driving piece 111A2 is fixedly connected to the second switching assembly 111H2, and the second lifting driving piece 111A2 is a motor. The second lifting transmission assembly 111E2 is arranged on the second switching assembly 111H2. The second lifting transmission assembly 111E2 is connected to the output end of the second lifting driving piece 111A2 and the second gripper 112B respectively.

The third lifting guide rail 111C3 is fixedly connected to the third switching assembly 111H3, the third lifting guide rail 111C3 extends along the second direction 20, and the third gripper 112C is slidably connected to the third lifting guide rail 111C3. The third lifting driving piece 111A3 is fixedly connected to the third switching assembly 111H3, and the third lifting driving piece 111A3 is a motor. The third lifting transmission assembly 111E3 is arranged on the third switching assembly 111H3. The third lifting transmission assembly 111E3 is connected to the output end of the third lifting driving piece 111A3 and the third gripper 112C respectively.

In the embodiment, the straight lines where the trajectories of the first gripper 112A and the second gripper 112B are located coincide, and the third gripper 112A is parallel to the straight lines where the trajectories of the first gripper 112A and the second gripper 112B are located. Since the number of access stations of the incubation device is equal to the number of non-coincident motion trajectories of the pick-and-place assembly 112 along the first direction 10, the number of access stations of the incubation device in the embodiment is two. That is, in the embodiment, the incubation device is provided with two access stations, namely, the first access station and the second access station. The first gripper 112A and the second gripper 112B move the reaction container 130 into and out of the incubation assembly 121 at the first access station, and the third gripper 112C moves the reaction container 130 into or out of the incubation assembly 121 at the second access station.

During operation, only one gripper moves the reaction container 130 in and out each time the incubation assembly 121 moves linearly along the third direction 30 to the access station to stop. The first gripper 112A, the second gripper 112B and the third gripper 112C move the reaction container 130 into and out of the incubation assembly 121 in a labor and time division manner. Specifically, the first gripper 112A moves the reactor that needs to be cleaned and separated out of the incubation assembly 121 and moves the cleaned and separated reaction container into the incubation assembly 121 each time the incubation assembly 121 moves linearly to the first access station to stop. The second gripper 112B moves the reactor that needs to be filled with the second reagent out of the incubation assembly 121, and moves the reaction container that needs to be incubated after being filled with the reagent into the incubation assembly 121. The third gripper 112C moves the reactor that needs to be measured after being moved and buffered by the incubation assembly 121 out of the incubation assembly 121 each time the incubation assembly 121 moves linearly along the third direction 30 to the second access station to stop. The first gripper 112A, the second gripper 112B and the third gripper 112C move the reaction container 130 in and out at different times, that is, only one gripper moves the reaction container 130 in and out each time the corresponding incubation assembly 121 moves linearly to the access station.

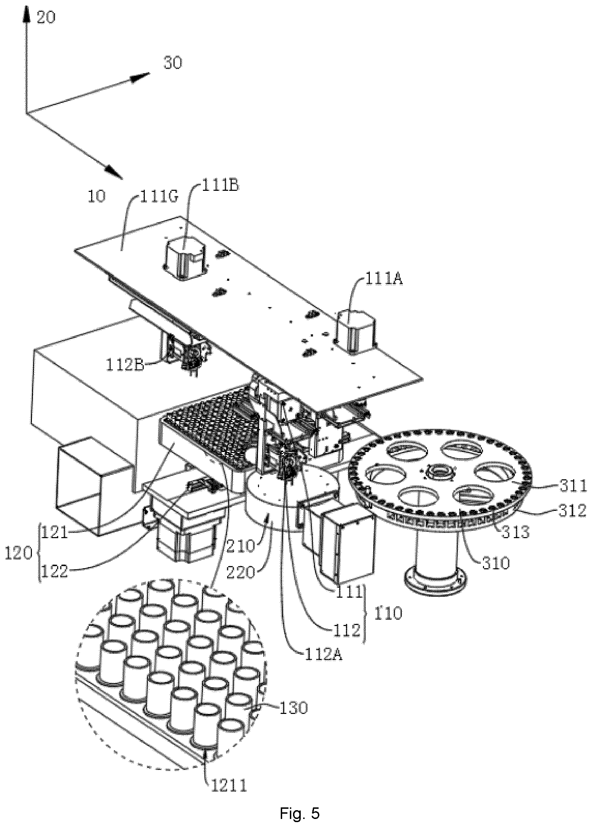

As shown in Fig. 5, the automatic analysis device further includes a cleaning and separation unit 310. The cleaning and separation unit 310 is non-nested with the incubation unit 120, that is, the cleaning and separation unit 310 and the incubation unit 120 are independently arranged, and the cleaning and separation unit 310 is configured to remove the unbound sample and reagent in the reaction container 130. Specifically, the cleaning and separation unit 310 is configured to clean and separate the reaction container 130 entering the cleaning and separation unit 310 and remove the unbound sample and reagent in the reaction container 130.

In one embodiment, the cleaning and separation unit 310 includes a rotary cleaning plate 311. Cleaning and separation positions 313 configured to carry the reaction containers 130 that need to be cleaned and separated are provided on the rotary cleaning plate 311. The cleaning and separation positions 313 are annularly arranged on the cleaning plate 311. At least 30 cleaning and separation positions 313 are provided. In this way, the processing efficiency of the cleaning and separation unit is guaranteed. During operation, the cleaning plate 311 rotates forward one cleaning and separation position 313 each time. A moving-out station and a moving-in station are provided on the cleaning and separation unit 310. The moving-out station is configured to move the reaction container 130 on the cleaning and separation position 313 out, and the moving-in station is configured to move the reaction container 130 into the cleaning and separation position 313. It is to be noted that, similar to the access station of the aforementioned incubation device, the moving-out station and the moving-in station on the cleaning and separation unit 310 are also absolute positions, which are configured to identify the absolute coordinate position relationship of the cleaning and separation position 313 on the cleaning plate 311 when the cleaning plate 311 works or performs a task, and does not move along with the rotation of the cleaning plate 311. The cleaning plate 311 rotates to position the reaction container 130 on each cleaning and separating position 313 to the moving-out station and the other one to the moving-in station. The moving-out station and the moving-in station are located under the motion trajectory of the first direction 10 of the pick-and-place assembly 112. Further, the moving-out station and the moving-in station are adjacent to each other. The moving-in station is located upstream of the moving-out station along the rotation direction of the rotary cleaning plate 311. Specifically, along the direction of rotating forward of the cleaning plate 311, the moving-out station is in the front, and the moving-out station is followed by the moving-out station. The cleaning plate 311 rotates forward to one cleaning and separation position 313 so as to position the reaction container 130 on the target cleaning and separation position 313 to the moving-out station. The pick-and-place assembly 112 moves along the first direction 10 to above the moving-out station so as to move the reaction container 130 on the target cleaning and separation position 313 out at the moving-out station. After the reaction container 130 is moved out, the target cleaning and separation position 313 is vacant, then the cleaning plate 311 rotates forward one cleaning and separation position 313 so as to position the vacant target cleaning and separation position 313 to the moving-in station. The pick-and-place assembly 112 moves along the first direction 10 to above the moving-in station so as to move the other reaction container 130 into the cleaning and separation position 313 at the moving-in station. In this way, the moving travel of the pick-and-place assembly 112 along the first direction 10 is saved, which facilitates the compactness of the analysis device and the improvement of the pick-and-place efficiency of the pick-and-place assembly 112.

The cleaning and separation unit 310 further includes a magnetic unit 312. The magnetic device 312 provides the magnetic force to collect the magnetic particles in the reaction container 130 to the inner wall of the reaction container 130. Due to the response time, moving distance, resistance and other factors in the magnetic field, it takes a certain time to collect the magnetic particles to the inner wall of the reaction container 130, usually ranging from a few seconds to tens of seconds. In this way, the reaction container 130 needs to pass through the magnetic field for a period of time before absorbing waste liquid (including unbound sample and reagent components). In the embodiment, the magnetic device 312 is directly installed or fixed near the reaction container 130 of the rotary cleaning plate 311, so that the magnetic device 312 is closer to the reaction container 130, which reduces the adsorption time of the magnetic particles and improves the cleaning and separation efficiency.

The cleaning and separation unit 310 further includes a washing mechanism. The washing mechanism is arranged above the cleaning and separation position 313, and includes a suction needle, a suction tube, a suction nozzle or other suction units suitable for sucking liquid. The suction unit is driven in and out of the reaction container 130 by a driving mechanism, and suck the unbound sample and reagent in the reaction container 130. The washing mechanism includes injection needle, tube, nozzle and other injection units suitable for discharging liquid, and a cleaning buffer solution is injected into the reaction container 130 after suction. Each washing includes a process of one-time suction and one-time injection of the cleaning buffer solution. Generally, washing three or four times, that is, washing is performed three or four times. Of course, the washing times are flexible. In order to make the cleaning more thorough and less residual, a mixer is arranged at the injection level to uniformly mix the reaction container 130 or use the impact force during liquid injection, and the magnetic particles are resuspended and evenly dispersed in the cleaning buffer solution at the same time or after the injection of the cleaning buffer solution.

In addition, the cleaning and separation unit 310 is further coupled with a signal reagent filling mechanism, after the reaction container 130 completes cleaning and separation, the signal reagent filling mechanism fills all or part of the signal reagent into the reaction container 130 through the cleaning and separation unit 130, for example, filling all first and second signal reagents or only filling the first signal reagent, and the rest of the signal reagent is filled during measurement.

As shown in Fig. 5, the automatic analysis device further includes a measuring unit 210. In one embodiment, the measuring unit 210 includes a rotary measuring plate 220, a measuring positions being provided on the rotary measuring plate 220 and configured to carry the reaction container 130 that needs to be measured. At least three measuring positions are provided. An access station is provided on the measuring unit 210 for the transfer unit 110 to move the reaction container 130 in and out. The access station on the measuring unit 210 is located under the motion trajectory of the first direction 10 of the pick-and-place assembly 112. The measuring plate 220 rotates to position the reaction container 130 on each measuring positions to the access station of measuring unit 210. It is to be noted that, similar to the moving-out station and the moving-in station of the aforementioned cleaning and separation unit 310, the access position on the measuring unit 210 is also an absolute position, which is configured to identify the absolute coordinate position relationship of the measuring positions on the measuring plate 220 when the measuring plate 220 works or performs a task, and does not move along with the rotation of the measuring plate 220. The measuring unit 210 further includes a pot body assembly, a pot cover assembly, a measuring assembly, etc. The measuring assembly includes a weak light detector Photomultiplier Tube (PMT) or other sensitive photoelectric induction devices, which convert a measured optical signal into an electrical signal and transmit it to a control center. In addition, in order to improve the measuring efficiency and ensure the measuring consistency, the measuring assembly further includes optical structures such as optical signal collection and calibration. The assembly of the measuring unit 210 is connected or installed to the pot body assembly in a general manner, such as directly installed and fixed to the pot body assembly or installed to the pot body assembly through optical fiber connection. The position where the measuring assembly is installed on the measuring unit 210 is a signal reading position, and the reaction container that contains the signal reagent completes the reading of the optical signal at the measuring position. In one embodiment, the measuring unit 210 further includes a temperature control assembly. The temperature control assembly includes an insulation material, a heater, a temperature sensor, etc., to provide a stable temperature environment for the measuring unit 210.

In another embodiment, the measuring unit 210 includes a linear driving mechanism, the linear driving mechanism being slidably connected to a carrier block, at least one measuring positions is provided on the carrier block to carry the reaction container 130 that needs to be measured. The measuring positions have at least two working positions, namely the first station and the second station. The first station is configured to move the transfer unit 110 in and out and the second station is configured for measurement. The two stations of the measuring unit 210 here are similar to the definition of the aforementioned station and will not be elaborated herein. The measuring assembly includes a weak light detector Photomultiplier Tube (PMT) or other sensitive photoelectric induction devices, which convert a measured optical signal into an electrical signal and transmit it to a control center. In addition, in order to improve the measuring efficiency and ensure the measuring consistency, the measuring assembly further includes optical structures such as optical signal collection and calibration.

In one embodiment, the cleaning and separation unit 310 and the measuring unit 210 are located on the same side of the incubation unit 120. In this way, the space on one side of the incubation unit 120 is fully utilized, which makes the structure of the automatic analysis unit more compact. The rotating center of the rotary cleaning plate 311 of the cleaning and separation unit 310 and the rotating center of the rotary measuring plate 220 of the measuring unit 210 are located on both sides of the motion trajectory of the pick-and-place assembly of the transfer unit 110 along the first direction 10 respectively. Specifically, the rotating center of the rotary cleaning plate 311 of the cleaning and separation unit 310 and the rotating center of the rotary measuring plate 220 of the measuring unit 210 are located on both sides of the motion trajectory of the first gripper 112A of the transfer unit 110 along the first direction 10 respectively. On the one hand, the moving travel of the transfer unit 110 along the first direction 10 is shorter, and on the other hand, the space on both sides of the first direction 10 is fully utilized, so that the automatic analysis device has a more compact structure and a higher space utilization.

The two-step method is used as an example to illustrate the working process of the automatic analysis device below. As shown in Fig. 3, the pick-and-place assembly 112 includes the first gripper 112A and the second gripper 112B as an example.

As shown in Fig. 5, the incubation assembly 121 moves linearly along the third direction 30 to the access station of the incubation device so as to convey the empty incubation position 1211 to the access position of the incubation device. The second gripper 112B moves along the first direction 10 to above the incubation assembly 121 (specifically, above the access position of the incubation device, the same below, will not be elaborated), and lifts along the second direction 20 so as to move the reaction container 130 that contains the sample and the first reagent into the incubation position 1211 of the incubation unit 120. The first incubation is performed on the sample and the first reagent in the reaction container 130 for a set time on the incubation assembly 121. Usually, after the first reaction, the reaction container 130 contains a magnetic particle complex formed by the binding of the sample and the first reagent. The incubation time is usually 3-60 minutes.

The incubation assembly 121 moves linearly along the third direction 30 to the access station of the incubation device so as to convey the reaction container 130 that needs the first cleaning and separation after the first incubation for a set time to the access station of the incubation device. The first gripper 112A moves along the first direction 10 to above the incubation assembly 121, and lifts along the second direction 20 so as to move the reaction container 130 out. The first gripper 112A moves along the first direction 10 to above the moving-in station on the cleaning and separation unit 310, the cleaning plate 311 rotates forward one cleaning and separation position 313 so as to position the empty cleaning and separation position 313 to the moving-in station. The first gripper 112A lifts along the second direction 20 to move the reaction container 130 into the cleaning and separation position 313.

The cleaning plate 311 rotates to drive the reaction container 130 forward in the cleaning and separation unit 310, and the first cleaning and separation is performed on the reaction container 130 to remove the unbound sample and reagent in the reaction container 130. The cleaning plate 311 rotates to position the reaction container 130 after the first cleaning and separation to the moving-out station on the cleaning and separation unit 310. The first gripper 112A moves along the first direction 10 to above the moving-out station on the cleaning and separation unit 310, and moves the reaction container 130 out along the second direction 20.

The incubation assembly 121 moves linearly along the third direction 30 to the access position of the incubation device so as to convey the empty incubation position 1211 to the access position of the incubation device. The first gripper 112A moves along the first direction 10 to above the incubation assembly 121, and lifts along the second direction 20 to move the reaction container 130 after the first cleaning and separation into the incubation position 1211 of the incubation unit 120. The reaction container 130 after the first cleaning and separation is buffered on the incubation position 1211 of the incubation unit 120.

The incubation assembly 121 moves linearly along the third direction 30 to the access position of the incubation device so as to convey the reaction container 130 that needs to be added with the second reagent after the first cleaning and separation to the access position of the incubation device. The second gripper 112B moves along the first direction 10 to above the incubation assembly 121, and lifts along the second direction 20 to move the reaction container 130 out of the incubation position 1211 of the incubation unit 120. The second reagent is added into the reaction container 130. Herein, the different second reagents are correspondingly selected according to different test items. According to the different second reagents and test methods, some test items also need to uniformly mix a reactant that contains the second reagent in the reaction container 130.

The incubation assembly 121 moves linearly along the third direction 30 to the access position of the incubation device so as to convey the empty incubation position 1211 to the access position of the incubation device. The second gripper 112B moves along the first direction 10 to above the incubation assembly 121, and lifts along the second direction 20 to move the reaction container 130 added with the second reagent into the incubation position 1211 of the incubation unit 120. The second incubation is performed on the magnetic particle complex after first cleaning and separation of the reaction container 130 and the second reagent in the reaction container 130 for the set time on the incubation assembly 121. Usually, the new magnetic particle complex is formed by binding in the reaction container 130 after the second reaction. The incubation time is usually 3-60 minutes.

The incubation assembly 121 moves linearly along the third direction 30 to the access position of the incubation device so as to convey the reaction container 130 that needs the second cleaning and separation after the second incubation for a set time to the access position of the incubation device. The first gripper 112A moves along the first direction 10 to above the incubation assembly 121, and lifts along the second direction 20 so as to move the reaction container 130 out. The first gripper 112A moves along the first direction 10 to above the moving-in station on the cleaning and separation unit 310, and the cleaning plate 311 rotates forward one cleaning and separation position 313 so as to position the empty cleaning and separation position 313 to the moving-in station. The first gripper 112A lifts along the second direction 20 to move the reaction container 130 into the cleaning and separation position 313.

The cleaning plate 311 rotates to drive the reaction container 130 forward in the cleaning and separation unit 310, and the second cleaning and separation is performed on the reaction container 130 to remove the unbound sample and reagent in the reaction container 130. After the reaction container 130 completes the second cleaning and separation, the signal reagent filling mechanism fills all or part of the signal reagent into the reaction container 130 through the cleaning and separation unit 130. The cleaning plate 311 rotates to position the reaction container 130 after the second cleaning and separation to the moving-out station on the cleaning and separation unit 310. The first gripper 112A moves along the first direction 10 to above the moving-out station on the cleaning and separation unit 310, and moves the reaction container 130 out along the second direction 20.

The incubation assembly 121 moves linearly along the third direction 30 to the access position of the incubation device so as to convey the empty incubation position 1211 to the access position of the incubation device. The first gripper 112A moves along the first direction 10 to above the incubation assembly 121, and lifts along the second direction 20 to move the reaction container 130 filled with the signal reagent after the second cleaning and separation into the incubation position 1211 of the incubation unit 120. The reaction container 130 filled with the signal reagent after the second cleaning and separation is buffered on the incubation position 1211 of the incubation unit 120. If the reaction container 130 requires signal incubation, signal incubation is performed on the incubation position 1211 of the incubation unit 120 for a set time.

The incubation assembly 121 moves linearly along the third direction 30 to the access position of the incubation device so as to convey the reaction container that needs to be measured after the second cleaning and separation to the access position of the incubation device. The second gripper 112B moves along the first direction 10 to above the incubation assembly 121, and lifts along the second direction 20 to move the reaction container 130 out of the incubation position 1211 of the incubation unit 120.

The second gripper 112B moves along the first direction 10 to above the access position of the measuring unit 210, and lifts along the second direction 20 to move the reaction container 130 into the measuring positions on the measuring plate 220. The measuring plate 220 rotates to position the reaction container 130 to a signal reading position, and the measuring assembly reads the optical signal in the reaction container 130.

According to the working process of the above automatic analysis device, the transfer unit 110 of the present invention is configured to move the reaction container 130 into and out of the incubation unit 120, and the incubation unit 120 is configured to incubate the reaction container 130 that contains the reactant or the buffered, cleaned and separated reaction container 130. The incubation assembly 121 moves linearly along the third direction 30 so as to convey the target incubation position 1211 to the access position of the incubation device. The pick-and-place driving assembly 111 drives the pick-and-place assembly 112 to move along the first direction 10 and the second direction 20 so as to move the reaction container 130 into or out of the incubation position 1211 at the access position of the incubation device. The rotating center of the rotary cleaning plate 311 of the cleaning and separation unit 310 and the rotating center of the rotary measuring plate 220 of the measuring unit 210 are located on both sides of the motion trajectory of the first gripper 112A of the transfer unit 110 along the first direction 10 respectively. The automatic analysis device of the application is compact in structure, low in manufacturing cost, and efficiently achieve high-flux continuous testing.

In the description of the present invention, it is to be understood that the orientations or positional relationships indicated by the terms "center", "longitudinal", "transverse", "length", "width", "thickness" "upper", "down", "front", "rear", "left", "right", "vertical", "horizontal", "top", "bottom", "interior", "exterior", "clockwise", "anticlockwise", "axial", "radial", "circumferential", etc. are based on the orientations or positional relationships shown in the drawings, and are only for the convenience of describing the present invention and simplifying the description. The description does not indicate or imply that the device or element referred to must have a specific orientation, be constructed and operated in a specific orientation, and therefore cannot be construed as limiting the present invention.

In addition, the terms "first" and "second" are used for descriptive purposes only, and cannot be understood as indicating or implying relative importance. Therefore, the features defined with "first" and "second" may explicitly or implicitly include at least one of the features. In the description of the present invention, "plurality" means at least two, such as two, three, etc., unless otherwise specifically defined.

In the present invention, the terms "install", "link", "connect", "set" should be broadly understood, unless otherwise specified and defined, for example, may be fixedly connected, or detachably connected, or integrally connected; may be mechanically connected, or electrically connected; and may be directly connected or indirectly connected through an intermediate medium, and may be the internal communication of two elements or the interaction relationship between two elements, unless otherwise limited. The specific meaning of the above-mentioned terminology in the present invention may be understood by those of ordinary skill in the art in specific circumstances.

In the present invention, unless otherwise specified and defined, the first feature "on" or "under" the second feature may be direct contact of the first and second features, or the first and second features may be in indirect contact through an intermediate medium. Moreover, the first feature "over", "above" and "on" the second feature may mean that the first feature is directly above or obliquely above the second feature, or it only means that the level of the first feature is higher than the second feature. The first feature "neath", "below" and "under" the second feature may mean that the first feature is directly below or obliquely below the second feature, or it only means that the level of the first feature is smaller than the second feature.

It is to be noted that when an element is referred to as being "fixed to" another element, it may be directly on another element or there may also be a centered element. When an element is considered to be "connected" to another element, it may be directly connected to another element or there may also be a centered element at the same time. The terms "vertical", "horizontal", "upper", "down", "left", "right" and similar expressions used herein are for illustrative purposes only and do not mean the unique implementation mode.

The technical features of the above-described embodiments may be arbitrarily combined. For the sake of brevity of description, all possible combinations of the technical features in the above embodiments are not described. However, as long as there is no contradiction between the combinations of these technical features, all should be considered as the scope of this description.

The above embodiments are merely illustrative of several implementation modes of the present invention with specific and detailed description, and are not to be construed as limiting the patent scope of the present invention. It is to be noted that a number of variations and modifications is made by those of ordinary skill in the art without departing from the conception of the present invention, and all fall within the scope of protection of the present invention. Therefore, the scope of protection of the present invention should be determined by the appended claims. Although the preferred embodiments of the present invention are described in detail above, it should be understood that many variations and modifications of the basic inventive concept described herein that are apparent to those skilled in the art fall within the spirit and scope of the present invention limited by the appended claims.

## Claims

1. An incubation device, comprising:
an incubation unit (120), configured to incubate reaction containers (130) that contain a reactant or to buffer the cleaned and separated reaction containers (130), the incubation unit (120) comprising an incubation assembly (121) and an incubation driving assembly (122), incubation positions (1211) configured to place the reaction containers (130) being provided on the incubation assembly (121), and the incubation driving assembly (122) being connected to the incubation assembly (121) so as to drive the incubation assembly (121) to move linearly along a third direction (30); and
a transfer unit (110), configured to move the reaction containers (130) into or out of the incubation unit (120), the transfer unit (110) comprising a pick-and-place assembly (112) and a pick-and-place driving assembly (111), and the pick-and-place driving assembly (111) being connected to the pick-and-place assembly (112) so as to drive the pick-and-place assembly (112) to move along a first direction (10) and a second direction (20);
wherein, when the pick-and-place assembly (112) moves along the first direction (10), same able to move to above the incubation assembly (121), and the pick-and-place assembly (112) moves along the second direction (20) so as to move the reaction containers (130) into or out of the incubation positions (1211), the first direction (10) and the third direction (30) are perpendicular, the first direction (10) and the second direction (20) are perpendicular.

2. The incubation device as claimed in claim 1, wherein the incubation positions (1211) are arranged in a matrix on the incubation assembly (121).

3. The incubation device as claimed in claim 1, wherein the pick-and-place assembly (112) comprises a first gripper (112A); the pick-and-place driving assembly (111) comprises:
a fixed plate (111G);
a first guide rail (111C), which is fixedly connected to the fixed plate (111G) and extends along the first direction (10), the first gripper (112A) being slidably connected to the first guide rail (111C);
a first driving piece (111A), which is fixedly connected to the fixed plate (111G); and
a first transmission assembly (111E), which is arranged on the fixed plate (111G), and is connected to an output end of the first driving piece (111A) and the first gripper (112A) respectively so as to enable the first driving piece (111A) to drive the first gripper (112A) to slide along the first guide rail (111C).

4. The incubation device as claimed in claim 3, wherein the pick-and-place assembly (112) comprises a second gripper (112B) slidably connected to the first guide rail (111C); the pick-and-place driving assembly (111) comprises:
a second driving piece (111B), which is fixedly connected to the fixed plate (111G); and
a second transmission assembly (111J), which is arranged on the fixed plate (111G), and is connected to an output end of the second driving piece (111B) and the second gripper (112B) respectively so as to enable the second driving piece (111B) to drive the second gripper (112B) to slide along the first guide rail (111C).

5. The incubation device as claimed in claim 4, wherein the pick-and-place assembly (112) comprises a third gripper (112C);
the pick-and-place driving assembly (111) comprises:
a second guide rail (111D), which is fixedly connected to the fixed plate (111G) and is parallel to the first guide rail (111C), the third gripper (112C) being slidably connected to the second guide rail (111D);
a third driving piece (111I), which is fixedly connected to the fixed plate (111G); and
a third transmission assembly (111F), which is arranged on the fixed plate (111G), and is connected to an output end of the third driving piece (111I) and the third gripper (112C) respectively so as to enable the third driving piece (111I) to drive the third gripper (112C) to slide along the second guide rail (111D).

6. The incubation device as claimed in claim 4, wherein a plurality of incubation positions (1211) are arranged and are at least arranged along the first direction (10); the incubation assembly (121) moves linearly along the third direction (30), and the first gripper (112A) isable to move along the first direction (10) to above all the incubation positions (1211) arranged along the first direction (10), and the incubation assembly (121) moves linearly along the third direction (30), and the second gripper (112B) isable to move along the first direction (10) to above all the incubation positions (1211) arranged along the first direction (10).

7. An automatic analysis device, comprising the incubation device as claimed in any one of claims 1-6.

8. An automatic analysis device, comprising:
an incubation unit (120), configured to incubate reaction containers (130) that contain a sample and a reagent, the sample and the reagent being combined after incubation, the incubation unit (120) comprising an incubation assembly (121) and an incubation driving assembly (122), incubation positions (1211) configured to place the reaction containers (130) being provided on the incubation assembly (121), and the incubation driving assembly (122) being connected to the incubation assembly (121) so as to drive the incubation assembly (121) to move linearly along a third direction (30);
a transfer unit (110), configured to move the reaction containers (130) into or out of the incubation unit (120), the transfer unit (110) comprising a pick-and-place assembly (112) and a pick-and-place driving assembly (111), the pick-and-place driving assembly (111) being connected to the pick-and-place assembly (112) so as to drive the pick-and-place assembly (112) to move along a first direction (10) and a second direction (20); and
a cleaning and separation unit (310), which is non-nested with the incubation unit (120), and is configured to remove an unbound sample and reagent in the reaction containers (130);
wherein, when the pick-and-place assembly (112) moves along the first direction (10), same able to move to above the incubation assembly (121), and the pick-and-place assembly (112) moves along the second direction (20) so as to move the reaction containers (130) into or out of the incubation positions (1211).

9. The automatic analysis device as claimed in claim 8, wherein the cleaning and separation unit (310) comprises a rotary cleaning plate (311), cleaning and separation positions (313) configured to carry the reaction containers (130) that need to be cleaned and separated being provided on the rotary cleaning plate (311), and the rotary cleaning positions (313) being annularly arranged on the cleaning plate (311).

10. The automatic analysis device as claimed in claim 9, wherein when the pick-and-place assembly (112) moves along the first direction (10), same able to move to a moving-in station and a moving-out station, the rotary cleaning positions (313) annularly arranged on the cleaning plate (311) are able to move to the corresponding moving-in station and the moving-out station so as to enable the pick-and-place assembly (112) to move the reaction container (130) into one rotary cleaning position (313) of the rotary cleaning positions (313) at the moving-in station and move the reaction container (130) out of another rotary cleaning position (313) of the rotary cleaning positions (313) at the moving-out station, the moving-out station and the moving-in station are adjacent to each other; and the moving-in station is located upstream of the moving-out station along the rotation direction of the rotary cleaning plate (311).

11. The automatic analysis device as claimed in claim 9, wherein the cleaning and separation unit (310) further comprises a magnetic unit (312), configured to provide the magnetic force to collect magnetic particles in the reaction container (130) in the rotary cleaning position (313) to an inner wall of the reaction container (130);
the cleaning and separation unit (310) further comprises a washing mechanism; the washing mechanism comprises:
a suction unit, configured to enter and exit the reaction container (130) so as to suck the unbound sample and reagent; and
an injection unit, configured to inject a cleaning buffer solution into the reaction container (130).

12. The automatic analysis device as claimed in claim 8, further comprising a measuring unit (210), the measuring unit (210) being non-nested with the incubation unit (120) and the cleaning and separation unit (310), and the measuring unit (210) comprising measuring positions configured to carry the reaction container (130) that needs to be measured; and an access position configured to move the reaction containers (130) in and out is provided on the measuring unit (210), the measuring unit (210) comprises a rotary measuring plate (220), the measuring positions being provided on the rotary measuring plate (220).

13. The automatic analysis device as claimed in claim 12, wherein when the pick-and-place assembly (112) moves along the first direction (10), same able to move to the access station, there are multiple access station, and the measuring positions on the rotating measuring plate (220) are all move corresponding to the access stations, so that the pick-and-place assembly (112) is able to move in ore move out from the reaction containers (130) at the measuring positions.

14. The automatic analysis device as claimed in claim 17, wherein the pick-and-place assembly (112) comprises a first gripper (112A); the pick-and-place driving assembly (111) comprises:
a fixed plate (111G);
a first guide rail (111C), which is fixedly connected to the fixed plate (111G) and extends along the first direction (10), the first gripper (112A) being slidably connected to the first guide rail (111C);
a first driving piece (111A), which is fixedly connected to the fixed plate (111G); and
a first transmission assembly (111E), which is arranged on the fixed plate (111G), and is connected to an output end of the first driving piece (111A) and the first gripper (112A) respectively so as to enable the first driving piece (111A) to drive the first gripper (112A) to slide along the first guide rail (111C), a rotating center of the rotary measuring plate (220) and a rotating center of the cleaning plate (311) are located on both sides of a motion trajectory of the first gripper (112A) along the second direction (20) respectively, the pick-and-place assembly (112) comprises a second gripper (112B) slidably connected to the first guide rail (111C); the pick-and-place driving assembly (111) comprises:
a second driving piece (111B), which is fixedly connected to the fixed plate (111G); and
a second transmission assembly (111J), which is arranged on the fixed plate (111G), and is connected to an output end of the second driving piece (111B) and the second gripper (112B) respectively so as to enable the second driving piece (111B) to drive the second gripper (112B) to slide along the first guide rail (111C).

15. The automatic analysis device as claimed in claim 14, wherein a rotating center of the rotary measuring plate (220) and a rotating center of the cleaning plate (311) are located on both sides of a motion trajectory of the pick-and-place assembly (112) along the first direction (10) respectively; or
a rotating center of the rotary measuring plate (220) and a rotating center of the cleaning plate (311) are located on the same side of the incubation assembly (121).
